# EUROPEAN PATENT APPLICATION

(11) **EP 4 424 757 A1**
(43) Date of publication of application: **04.09.2024**
(21) Application number: 22886761.0
(22) Date of filing: 17.10.2022
(51) Int. Cl.: C08J 11/10, B29B 17/00, C07D 201/12, B09B 3/00

(54) **METHOD FOR RECOVERING FIBROUS FILLER AND EPSILON-CAPROLACTAM, AND METHOD FOR PRODUCING POLYAMIDE 6**

(30) Priority: 29.10.2021 JP 2021178280
(71) Applicant: Toray Industries, Inc., Tokyo 103-8666 (JP)
(72) Inventor: YAMASHITA, Kohei, Nagoya-shi, Aichi 455-8502 (JP); TAKAHASHI, Akihiro, Nagoya-shi, Aichi 455-8502 (JP); KATO, Masashi, Tokai-shi, Aichi 476-8567 (JP); NISHIMURA, Mihoko, Nagoya-shi, Aichi 455-8502 (JP); KATO, Koya, Nagoya-shi, Aichi 455-8502 (JP)
(74) Representative: Mewburn Ellis LLP
(86) International application number: PCT/JP2022/038577
(87) International publication number: WO 2023/074433

(57) **Abstract**

To provide a method for collecting a fibrous filling material and ε-caprolactam with high purity only by solid-liquid separation from a polyamide 6 resin composition containing a fibrous filling material. The present invention is a method for collecting a fibrous filling material and ε-caprolactam, including adding a polyamide 6 resin composition (A) containing at least a fibrous filling material (D) and at least one of water (B) heated to 290°C or higher and 350°C or lower and a polyamide 6 oligomer aqueous solution (B1) heated to 290°C or higher and 350°C or lower to make contact with each other to obtain a mixture (C) containing at least the fibrous filling material (D), ε-caprolactam, a polyamide 6 oligomer, and the water (B), and collecting the fibrous filling material (D) and the ε-caprolactam from the mixture (C), wherein steps (a) to (c) below are performed in this order:
(a) a step of preparing the mixture (C) by adding the polyamide 6 resin composition (A) containing the fibrous filling material (D) and at least one of the water (B) and the polyamide 6 oligomer aqueous solution (B1) to make contact with each other under a condition that a product of X and Y is 2,000 or less when a mass ratio of water to polyamide 6 or a mass ratio of water to a sum of polyamide 6 and the polyamide 6 oligomer is represented by X : 1 and a reaction temperature is represented by Y°C;
(b) a step of subjecting the mixture (C) to a solid-liquid separation (I) in a temperature range not higher than a boiling point of water at an operation pressure; and
(c) a step of washing a filter residue containing the fibrous filling material (D) obtained by the solid-liquid separation (I) with water having a temperature not higher than the boiling point of water at normal pressure to collect the fibrous filling material (D).

## Description

### Field

The present invention relates to a method for collecting a fibrous filling material and ε-caprolactam by depolymerizing a fiber-reinforced polyamide resin composition, which achieves both circulation utilization of fossil resources and reduction in global warming gas emissions, and more particularly to a recycling method for collecting a fibrous filling material and ε-caprolactam with high purity in high yield by depolymerizing a fiber-reinforced polyamide 6 resin composition using a small amount of water having a high specific heat capacity and a high vaporization heat.

### Background

In recent years, with the ocean plastic problem as a trigger, an interest in global environmental issues has increased, and there is a growing recognition that construction of a sustainable society is required. Global environmental issues include global warming, resource depletion, water shortage, and the like, and most of them are caused by an increase in resource consumption and global warming gas emissions due to rapid human activities after the industrial revolution. Therefore, in order to construct a sustainable society, technologies related to circulation utilization of fossil resources such as plastics and reduction in global warming gas emissions are increasingly important.

As a plastic recycling technology, a technology for conversion into a pyrolysis oil or gas by thermally decomposing a plastic waste material and collecting a gas, an oil, or the like has attracted attention, and many methods have been proposed. For example, Patent Literature 1 discloses a method for producing a hydrocarbon by a process including thermal decomposition and steam cracking of a waste plastic. These methods have an advantage that mixed waste plastics can be converted into a pyrolysis oil but cracking at a high temperature of 800°C or higher is required in order to convert a pyrolysis oil into a secondary raw material such as a plastic monomer, and further, when, for example, a plastic containing chlorine such as polyvinyl chloride or sulfur such as polyarylene sulfide is mixed in waste plastics, there is a concern about plant corrosion, and when a plastic containing oxygen and nitrogen such as a polyamide is mixed therein, there is a concern about explosion.

As a method for recycling polyamide 6, which is used in a large amount in various fields as a fiber, a film, and an engineering plastic, a method for obtaining ε-caprolactam, which is a raw material, by blowing superheated steam in the presence of a phosphoric acid catalyst is disclosed (see, for example, Patent Literature 2) .

In addition, as a method for depolymerizing polyamide 6 without using a catalyst such as an acid or a base, a method for collecting a lactam by bringing polyamide 6 and superheated water into contact with each other at a temperature of 280°C to 320°C is disclosed (see, for example, Patent Literatures 3 and 4.).

### Citation List

### Patent Literature

Patent Literature 1: JP 2019-533041 A
Patent Literature 2: JP H08-217746 A
Patent Literature 3: JP H10-510280 A
Patent Literature 4: JP H10-510282 A

### Summary

### Technical Problem

The method for collecting ε-caprolactam disclosed in Patent Literature 2 is a high-yield reaction in which the depolymerization yield of polyamide 6 is 80% or more, but requires a long time for the depolymerization reaction. Further, since a large amount of superheated water vapor, which is about 10 times the amount of polyamide 6 fibers, is required, the technology still has a problem in achieving both circulation utilization of fossil resources and reduction in global warming gas emissions. In addition, since this method is a reaction using phosphoric acid as a catalyst, the reaction is susceptible to impurities such as catalyst deactivation due to an additive contained in a plastic or adhering impurities in a waste plastic. In fact, it has been found that when the present inventors have conducted a collection experiment using polyamide 6 containing a potassium salt as a raw material under the same or similar conditions to the method described in Patent Literature 2, the yield significantly decreases. This is considered to be due to deactivation of the phosphoric acid catalytic action by the potassium salt. In addition, Patent Literature 2 does not disclose a method for collecting a fibrous filling material with high purity having a small amount of organic substances adhered thereto.

Meanwhile, in the methods for collecting ε-caprolactam disclosed in Patent Literatures 3 and 4, only water is used in the depolymerization reaction, and a catalyst such as phosphoric acid is not used, so that there is an advantage that reaction deactivation due to an additive, adhering impurities, or the like does not occur. However, in the disclosed method for collecting ε-caprolactam, water having a specific heat capacity of 4.2 kJ/kg·K and a vaporization heat of 2,250 kJ/kg, which are very high, is used in an amount about 10 times the amount of polyamide 6, which is large, to carry out a reaction for a long time, and thus a large amount of energy is required in the depolymerization reaction and the collection of ε-caprolactam from a low concentration ε-caprolactam aqueous solution. In addition, even when the amount of water used was simply reduced under the same conditions or similar conditions, the collection rate of ε-caprolactam only decreased. This is considered to be because the thermodynamic equilibrium point of ε-caprolactam generated by depolymerization and a linear oligomer generated by hydrolytic ring-opening of ε-caprolactam moved to the linear oligomer side only by simply reducing the amount of water used. In addition, Patent Literatures 3 and 4 do not disclose collection of a fibrous filling material with high purity having a small amount of organic substances adhered thereto.

### Solution to Problem

In order to solve the above problems, the present invention has the following configurations.
1. A method for collecting a fibrous filling material and ε-caprolactam, including
   adding a polyamide 6 resin composition (A) containing at least a fibrous filling material (D) and at least one of water (B) heated to 290°C or higher and 350°C or lower and a polyamide 6 oligomer aqueous solution (B1) heated to 290°C or higher and 350°C or lower to make contact with each other to obtain a mixture (C) containing at least the fibrous filling material (D), ε-caprolactam, a polyamide 6 oligomer, and the water (B), and
   collecting the fibrous filling material (D) and the ε-caprolactam from the mixture (C), wherein steps (a) to (c) below are performed in this order:
      (a) a step of preparing the mixture (C) by adding the polyamide 6 resin composition (A) containing the fibrous filling material (D) and at least one of the water (B) and the polyamide 6 oligomer aqueous solution (B1) to make contact with each other under a condition that a product of X and Y is 2,000 or less when a mass ratio of water to polyamide 6 or a mass ratio of water to a sum of polyamide 6 and the polyamide 6 oligomer is represented by X : 1 and a reaction temperature is represented by Y°C;
      (b) a step of subjecting the mixture (C) to a solid-liquid separation (I) in a temperature range not higher than a boiling point of water at an operation pressure; and
      (c) a step of washing a filter residue containing the fibrous filling material (D) obtained by the solid-liquid separation (I) with water having a temperature not higher than the boiling point of water at normal pressure to collect the fibrous filling material (D).
2. The method for collecting a fibrous filling material and ε-caprolactam according to item 1, in which the polyamide 6 resin composition (A) containing at least the fibrous filling material (D) and the water (B) heated to 290°C or higher and 350°C or lower, or, the water (B) heated to 290°C or higher and 350°C or lower and the polyamide 6 oligomer aqueous solution (B1) heated to 290°C or higher and 350°C or lower are added to make contact with each other to obtain the mixture (C) containing at least the fibrous filling material (D), the ε-caprolactam, the polyamide 6 oligomer, and the water (B), and then the fibrous filling material (D) and the ε-caprolactam are collected from the mixture (C), wherein steps (a) to (c) below are performed in this order:
   (a) a step of preparing the mixture (C) by adding the polyamide 6 resin composition (A) containing the fibrous filling material (D) and the water (B), or by adding the polyamide 6 resin composition (A) containing the fibrous filling material (D), the water (B), and the polyamide 6 oligomer aqueous solution (B1), to make contact with each other under a condition that a product of X and Y is 2,000 or less when a mass ratio of water to polyamide 6 or a mass ratio of water to a sum of polyamide 6 and the polyamide 6 oligomer is represented by X : 1 and a reaction temperature is represented by Y°C;
   (b) a step of subjecting the mixture (C) to solid-liquid separation (I) in a temperature range not higher than a boiling point of water at an operation pressure; and
   (c) a step of washing a filter residue containing the fibrous filling material (D) obtained by the solid-liquid separation (I) with water having a temperature not higher than the boiling point of water at normal pressure to collect the fibrous filling material (D).
3. The method for collecting a fibrous filling material and ε-caprolactam according to item 1 or 2, wherein a mass loss of the fibrous filling material (D) when the fibrous filling material (D) collected is subjected to a heating treatment in an air atmosphere at 600°C for 3 hours is 3.0 mass% or less.
4. The method for collecting a fibrous filling material and ε-caprolactam according to any one of items 1 to 3, wherein the solid-liquid separation (I) is performed in a temperature range not higher than the boiling point of water at normal pressure.
5. The method for collecting a fibrous filling material and ε-caprolactam according to any one of items 1 to 4, wherein the mixture (C) containing at least the fibrous filling material (D), the ε-caprolactam, the polyamide 6 oligomer, and water is prepared, and then subsequently subjected to the solid-liquid separation (I).
6. The method for collecting a fibrous filling material and ε-caprolactam according to any one of items 1 to 5, wherein the polyamide 6 oligomer aqueous solution (B1) is an extract liquid obtained in a step of extracting a polyamide 6 oligomer with hot water from polyamide 6 which is a product at a time of production of polyamide 6.
7. The method for collecting a fibrous filling material and ε-caprolactam according to any one of items 1 to 6, wherein the polyamide 6 resin composition (A) containing at least the fibrous filling material (D) is a waste of a resin molded body containing polyamide 6 including at least the fibrous filling material (D).
8. A method for producing polyamide 6, comprising obtaining ε-caprolactam by the method according to any one of items 1 to 7, and polymerizing polyamide 6.

### Advantageous Effects of Invention

The present invention can provide a collection method capable of collecting ε-caprolactam and a fibrous filling material with high purity in high yield with a small energy consumption even when a small amount of water having a high specific heat capacity is used in a method for collecting ε-caprolactam and a fibrous filling material by depolymerizing a polyamide 6 resin composition containing a fibrous filling material.

### Description of Embodiments

The present invention is a method, in which a polyamide 6 resin composition (A) containing at least a fibrous filling material (D) and at least one of water (B) heated to 290°C or higher and 350°C or lower and a polyamide 6 oligomer aqueous solution (B1) heated to 290°C or higher and 350°C or lower are added to make contact with each other to obtain a mixture (C) containing at least the fibrous filling material (D), ε-caprolactam, a polyamide 6 oligomer, and water, and then the fibrous filling material (D) and ε-caprolactam are collected from the mixture (C), and is characterized in that steps (a) to (c) below are performed in this order.
(a) a step of preparing the mixture (C) by adding the polyamide 6 resin composition (A) containing the fibrous filling material (D) and at least one of the water (B) and the polyamide 6 oligomer aqueous solution (B1) to make contact with each other under a condition that a product of X and Y is 2,000 or less when a mass ratio of the water (B) to polyamide 6 or a mass ratio of water to a sum of polyamide 6 and the polyamide 6 oligomer is represented by X : 1 and a reaction temperature is represented by Y°C
(b) a step of subjecting the mixture (C) to a solid-liquid separation (I) in a temperature range not higher than a boiling point of water at an operation pressure
(c) a step of washing a filter residue containing the fibrous filling material (D) obtained by the solid-liquid separation (I) with water having a temperature not higher than the boiling point of water at normal pressure to collect the fibrous filling material (D)

Hereinafter, the present invention will be described in more detail.

### (1) Polyamide 6 resin composition (A)

The polyamide 6 used in the present invention is a polyamide resin in which 6-aminocaproic acid and/or ε-caprolactam is used as a main raw material. The polyamide 6 may be one obtained by copolymerization with another monomer as long as the object of the present invention is not impaired. Here, the phrase "used as a main raw material" means that a total of 50 mol% or more of a unit derived from 6-aminocaproic acid or a unit derived from ε-caprolactam is contained in a total of 100 mol% of monomer units included in the polyamide resin. A unit derived from 6-aminocaproic acid or a unit derived from ε-caprolactam is more preferably contained in an amount of 70 mol% or more, and still more preferably 90 mol% or more.

Examples of another monomer to be copolymerized include amino acids such as 11-aminoundecanoic acid, 12-aminododecanoic acid, and para-aminomethylbenzoic acid, lactams such as ω-laurolactam, aliphatic diamines such as tetramethylenediamine, pentamethylenediamine, hexamethylenediamine, 2-methylpentamethylenediamine, nonamethylenediamine, decamethylenediamine, undecamethylenediamine, dodecamethylenediamine, 2,2,4-/2,4,4-trimethylhexamethylenediamine, and 5-methylnonamethylenediamine, aromatic diamines such as meta-xylylenediamine and para-xylylenediamine, alicyclic diamines such as 1,3-bis(aminomethyl)cyclohexane, 1,4-bis(aminomethyl)cyclohexane, 1-amino-3-aminomethyl-3,5,5-trimethylcyclohexane, bis(4-aminocyclohexyl)methane, bis(3-methyl-4-aminocyclohexyl)methane, 2,2-bis(4-aminocyclohexyl)propane, 1,4-bis(3-aminopropyl)piperazine, and 1-(2-aminoethyl)piperazine, aliphatic dicarboxylic acids such as adipic acid, suberic acid, azelaic acid, sebacic acid, and dodecanedioic acid, aromatic dicarboxylic acids such as terephthalic acid, isophthalic acid, 2-chloroterephthalic acid, 2-methylterephthalic acid, 5-methylisophthalic acid, 5-sodium sulfoisophthalate, and 2,6-naphthalenedicarboxylic acid, and alicyclic dicarboxylic acids such as 1,4-cyclohexanedicarboxylic acid, 1,3-cyclohexanedicarboxylic acid, 1,2-cyclohexanedicarboxylic acid, and 1,3-cyclopentanedicarboxylic acid. Two or more of these may be copolymerized.

In addition, a polymerization degree regulator, a terminal group regulator or the like may be added to such polyamide 6. Examples of the polymerization degree regulator and the terminal group regulator include acetic acid and benzoic acid.

The polymerization degree of the polyamide 6 of the present invention is not particularly limited, but the relative viscosity measured at 25°C in a 98% concentrated sulfuric acid solution having a resin concentration of 0.01 g/mL is preferably in a range of 1.5 to 5.0. The relative viscosity in such a preferable range can be preferably exemplified because the reaction efficiency with a small amount of water tends to increase.

The polyamide 6 resin composition (A) of the present invention contains a fibrous filling material (D). Specific examples of the fibrous filling material (D) here include glass fibers, glass flat fibers, modified cross-section glass fibers, glass cut fibers, flat glass fibers, and carbon fibers, and two or more types of these may be used in combination. Among them, glass fibers are preferable. The content of the fibrous filling material (D) in the resin composition (A) is preferably 1 to 200 parts by mass with respect to 100 parts by mass of the resin composition (A).

In the resin composition (A) of the present invention, a filler, a thermoplastic resin other than polyamide 6, various additives, or the like can be further blended as long as the object of the present invention is not impaired.

The filler may be either an organic filler or an inorganic filler, and examples thereof include non-fibrous filling materials, and two or more of these may be blended. Examples of the non-fibrous filling material include non-swellable silicates such as talc, wollastonite, zeolite, sericite, mica, kaolin, clay, pyrophyllite, bentonite, asbestos, alumina silicate, and calcium silicate, swellable layered silicates such as Li-type fluoroteniolite, Na-type fluoroteniolite, Na-type tetrasilicon fluorine mica, and Li-type tetrasilicon fluorine mica, metal oxides such as silicon oxide, magnesium oxide, alumina, silica, diatomaceous earth, zirconium oxide, titanium oxide, iron oxide, zinc oxide, calcium oxide, tin oxide, and antimony oxide, metal carbonates such as calcium carbonate, magnesium carbonate, zinc carbonate, barium carbonate, dolomite, and hydrotalcite, metal sulfates such as calcium sulfate and barium sulfate, metal hydroxides such as magnesium hydroxide, calcium hydroxide, aluminum hydroxide, and basic magnesium carbonate, smectite-based clay minerals such as montmorillonite, beidellite, nontronite, saponite, hectorite, and sauconite, various clay minerals such as vermiculite, halloysite, kanemite, kenyaite, zirconium phosphate, and titanium phosphate, glass beads, glass flakes, ceramic beads, boron nitride, aluminum nitride, silicon carbide, calcium phosphate, carbon black, and graphite. In the swellable layered silicate described above, an exchangeable cation present between layers may be exchanged with an organic onium ion. Examples of the organic onium ion include an ammonium ion, a phosphonium ion, and a sulfonium ion.

Specific examples of the various additives include a heat stabilizer such as a phenolic compound such as N,N'-hexamethylenebis(3,5-di-t-butyl-4-hydroxyhydrocinnamide) or tetrakis[methylene-3-(3',5'-di-t-butyl-4'-hydroxyphenyl)propionate]methane, a phosphorus-based compound, a sulfur-based compound such as a mercaptobenzimidazole-based compound, a dithiocarbamic acid-based compound, or an organic thioacid-based compound, or an amine-based compound such as N,N'-di-2-naphthyl-p-phenylenediamine or 4,4'-bis(α,α-dimethylbenzyl)diphenylamine, a coupling agent such as an isocyanate-based compound, an organic silane-based compound, an organic titanate-based compound, an organic borane-based compound, or an epoxy compound, a plasticizer such as a polyalkylene oxide oligomer-based compound, a thioether-based compound, an ester-based compound, or an organic phosphorus-based compound, a crystal nucleating agent such as an organic phosphorus compound or polyether ether ketone, a metal soap such as a montanic acid wax, lithium stearate, or aluminum stearate, a release agent such as ethylenediamine-stearic acid-sebacic acid polycondensate or a silicone-based compound, an anti-coloring agent such as a hypophosphite, a lubricant, an ultraviolet light inhibitor, a colorant, a flame retardant, and a foaming agent. When such an additive is contained, the content thereof is preferably 10 parts by mass or less, and more preferably 1 part by mass or less with respect to 100 parts by mass of polyamide 6.

Specific examples of the thermoplastic resin other than polyamide 6 contained in the polyamide 6 resin composition (A) include a polyamide resin other than polyamide 6, a polyester resin, a polyolefin resin, a modified polyphenylene ether resin, a polysulfone resin, a polyketone resin, a polyetherimide resin, a polyarylate resin, a polyethersulfone resin, a polyetherketone resin, a polythioetherketone resin, a polyetheretherketone resin, a polyimide resin, a polyamideimide resin, and a tetrafluorinated polyethylene resin. Two or more of these may be blended. Further, it can be preferably exemplified that the blending amount of the thermoplastic resin other than polyamide 6 here is set to 30 parts by mass or less with respect to 100 parts by mass of polyamide 6 in the polyamide 6 resin composition (A) of the present invention.

The polyamide 6 resin composition (A) containing at least the fibrous filling material (D) of the present invention may be a waste of a resin molded body containing polyamide 6 including at least the fibrous filling material (D). Examples of the waste of the resin molded body include a polyamide 6 product, an industrial waste generated in the process for producing a polyamide 6 product, and a used polyamide 6 product waste. Examples of the polyamide 6 product including the fibrous filling material (D) include molded parts for residential building materials, electrical and electronic molded parts, aircraft parts, industrial machine parts, extrusion molded products, in situ polymerized molded products, and RIM molded products. In addition, product scraps, pellet scraps, block scraps, cutting scraps during cutting work, and the like generated in these production steps also serve as waste targets.

### (2) Polyamide 6 oligomer

The polyamide 6 oligomer in the present invention is a polyamide 6 oligomer in which 6-aminocaproic acid and/or ε-caprolactam is contained as a main component. The polyamide 6 oligomer may contain another monomer as long as the object of the present invention is not impaired. The phrase "contained as a main component" here means that a total of 50 mol% or more of a unit derived from 6-aminocaproic acid or a unit derived from ε-caprolactam is contained in a total of 100 mol% of monomer units included in the polyamide 6 oligomer. A unit derived from 6-aminocaproic acid or a unit derived from ε-caprolactam is more preferably contained in an amount of 70 mol% or more, and still more preferably 90 mol% or more.

Examples of another monomer contained in the polyamide 6 oligomer include amino acids such as 11-aminoundecanoic acid, 12-aminododecanoic acid, and para-aminomethylbenzoic acid, lactams such as ω-laurolactam, aliphatic diamines such as tetramethylenediamine, pentamethylenediamine, hexamethylenediamine, 2-methylpentamethylenediamine, nonamethylenediamine, decamethylenediamine, undecamethylenediamine, dodecamethylenediamine, 2,2,4-/2,4,4-trimethylhexamethylenediamine, and 5-methylnonamethylenediamine, aromatic diamines such as meta-xylylenediamine and para-xylylenediamine, alicyclic diamines such as 1,3-bis(aminomethyl)cyclohexane, 1,4-bis(aminomethyl)cyclohexane, 1-amino-3-aminomethyl-3,5,5-trimethylcyclohexane, bis(4-aminocyclohexyl)methane, bis(3-methyl-4-aminocyclohexyl)methane, 2,2-bis(4-aminocyclohexyl)propane, 1,4-bis(3-aminopropyl)piperazine, and 1-(2-aminoethyl)piperazine, aliphatic dicarboxylic acids such as adipic acid, suberic acid, azelaic acid, sebacic acid, and dodecanedioic acid, aromatic dicarboxylic acids such as terephthalic acid, isophthalic acid, 2-chloroterephthalic acid, 2-methylterephthalic acid, 5-methylisophthalic acid, 5-sodium sulfoisophthalate, and 2,6-naphthalenedicarboxylic acid, and alicyclic dicarboxylic acids such as 1,4-cyclohexanedicarboxylic acid, 1,3-cyclohexanedicarboxylic acid, 1,2-cyclohexanedicarboxylic acid, and 1,3-cyclopentanedicarboxylic acid. Two or more of these may be contained.

The number average molecular weight of the polyamide 6 oligomer of the present invention is not particularly limited, but it can be exemplified that the number average molecular weight is preferably in a range of 100 to 5,000, more preferably in a range of 200 to 3,000, and particularly preferably in a range of 200 to 2,000. When the molecular weight of the polyamide 6 oligomer is in such a preferable range, the solubility in water is increased, and a polyamide 6 oligomer aqueous solution (B1) used in the present invention tends to be easily prepared. The number average molecular weight here was calculated by GPC analysis using 1,1,1,3,3,3-hexafluoro-2-propanol as a solvent. GPC-HFIP-805 manufactured by Showa Denko K.K. was used as a column, and PMMA was used as a standard substance.

### (3) Method for preparing mixture (C)

In the present invention, a mixture (C) containing at least a fibrous filling material (D), ε-caprolactam, a polyamide 6 oligomer, and water is prepared by adding a polyamide 6 resin composition (A) containing at least the fibrous filling material (D) and at least one of water (B) heated to 290°C or higher and 350°C or lower and a polyamide 6 oligomer aqueous solution (B1) heated to 290°C or higher and 350°C or lower to make contact with each other. The present invention is characterized in that the mixture (C) is prepared under a condition that a product of X and Y is 2,000 or less when the mass ratio of the water (B) to polyamide 6 or water to the sum of polyamide 6 and the polyamide 6 oligomer is represented by X : 1 and a reaction temperature is represented by Y°C.

The water (B) used here is not particularly limited, and any water such as tap water, ion exchanged water, distilled water, or well water may be used, but ion exchanged water or distilled water is preferably used from the viewpoint of preventing side reactions due to the influence of coexisting salts.

In the present invention, water heated to 290°C or higher and 350°C or lower and water in the polyamide 6 oligomer aqueous solution (B1) heated to 290°C or higher and 350°C or lower are a reaction substrate. When the pressure is increased to 22.1 MPa and the temperature is increased to 374.2°C, water shows a state of being not a liquid or a gas. This point is referred to as a critical point of water, and hot water having a temperature and a pressure lower than the critical point is referred to as subcritical water. The water (B) or the polyamide 6 oligomer aqueous solution (B1) used in the present invention has a temperature of 290°C or higher and 350°C or lower, and corresponds to subcritical water. Although the subcritical water is water, the subcritical water has characteristics that (i) the permittivity is low and (ii) the ion product is high, and the permittivity and the ion product of the subcritical water depend on the temperature and the partial pressure of water and can be controlled. Due to its low permittivity, the subcritical water serves as an excellent solvent for an organic compound even though it is water, and due to its high ion product, the hydrogen ion and hydroxide ion concentrations are increased, so that the subcritical water has an excellent hydrolysis action. The temperature of the water (B) or the polyamide 6 oligomer aqueous solution (B1) of the present invention is preferably 300°C or higher and 340°C or lower, and more preferably 320°C or higher and 340°C or lower. When the temperature is in such a preferable range, there is a tendency that device corrosion during reaction can be prevented. Further, the pressure of the water (B) or the polyamide 6 oligomer aqueous solution (B1) can be preferably exemplified by a pressure higher than the saturated vapor pressure. As the water, water in a liquid state or water in a gas state such as water vapor or both may be used, but the pressure of the water (B) or the polyamide 6 oligomer aqueous solution (B1) is preferably higher than the saturated vapor pressure because the reaction is more likely to proceed in a liquid state than in a gas state in a reaction field. Further, the upper limit of the pressure of the water (B) or the polyamide 6 oligomer aqueous solution (B1) is not particularly limited, but can be exemplified by 20 MPa or less. Such a pressure range is preferable because the ion product of the water tends to increase. In order to bring the water into such a pressure range, a method of pressurizing the inside of a pressure vessel and sealing the pressure vessel can be mentioned. In order to pressurize the inside of the pressure vessel, a gas may be enclosed in addition to the water (B) or the polyamide 6 oligomer aqueous solution (B1), and examples of such a gas include air, argon, and nitrogen, but it is preferable to use nitrogen or argon from the viewpoint of preventing side reactions such as an oxidation reaction. The degree of gas pressurization is not particularly limited because it is set to achieve a target pressure, but may be 0.3 MPa or more.

The present invention is characterized in that the product of X and Y satisfies the condition of 2,000 or less when the mass ratio of the water (B) to polyamide 6 when the mixture (C) is prepared, the mass ratio of the sum of the water (B) and water in the polyamide 6 oligomer aqueous solution (B1) to the sum of polyamide 6 and the polyamide 6 oligomer, or the mass ratio of water in the polyamide 6 oligomer aqueous solution (B1) to the sum of polyamide 6 and the polyamide 6 oligomer is represented by X : 1, and the reaction temperature is represented by Y°C. It can be exemplified that the product of X and Y preferably satisfies the condition of 1,600 or less, more preferably satisfies the condition of 1,300 or less, and particularly preferably satisfies the condition of 1,200 or less. Further, the lower limit of the product of X and Y is not particularly limited, but can be exemplified by the condition of preferably 300 or more, more preferably the condition of 320 or more, and particularly preferably the condition of 340 or more. The present invention relates to a method for collecting ε-caprolactam and a fibrous filling material with high purity in an energy saving manner from a polyamide 6 resin composition including a fibrous filling material aiming at achieving both circulation utilization of fossil resources and reduction in global warming gas emissions. Since water has a specific heat capacity of 4.3 kJ/kg·K and a vaporization heat of 2,250 kJ/kg, which are very high as compared with other organic solvents, it is important to reduce the amount of water used, and when the product of X and Y is in such a condition range, it is possible to achieve both production efficiency of ε-caprolactam and energy saving. Further, when the retention time at the reaction temperature Y°C is represented by Z minutes, the product of X, Y, and Z can be preferably exemplified by the condition of 60,000 or less. The product of X, Y, and Z more preferably satisfies the condition of 40,000 or less, still more preferably the condition of 30,000 or less, and particularly preferably the condition of 20,000 or less. Further, the lower limit of the product of X, Y, and Z is not particularly limited, but can be exemplified by preferably the condition of 5,000 or more, more preferably the condition of 8,000 or more, and particularly preferably the condition of 9,000 or more. The product of X, Y, and Z in such a preferable condition range is preferable because the production efficiency of ε-caprolactam in an energy saving manner tends to increase. In the reaction of polyamide 6 with water, in addition to the production of ε-caprolactam, a side reaction of linear oligomer production by the reaction of ε-caprolactam with water proceeds as a side reaction, and when the amount of water used is simply reduced, a large amount of linear oligomers are produced, and therefore the production efficiency of ε-caprolactam is greatly reduced. As a result of elucidating the thermodynamic equilibrium point of the production reaction of ε-caprolactam by the reaction of polyamide 6 with water and the side reaction of linear oligomer production, the present inventors have found that by setting the product of X and Y and the product of X, Y, and Z within the above condition ranges, by-production of linear oligomers is prevented, and the production efficiency of ε-caprolactam is greatly improved, leading to the present invention.

In addition, it can be mentioned as a preferable aspect that the mixture (C) is prepared by further adding the polyamide 6 oligomer aqueous solution (B1) heated to 290°C or higher and 350°C or lower to the polyamide 6 resin composition (A) containing at least the fibrous filling material (D) and the water (B) heated to 290°C or higher and 350°C or lower to make contact with each other.

The structure of the polyamide 6 oligomer in the polyamide 6 oligomer aqueous solution (B1) is similar to that described in the above-mentioned item (2). Further, the composition of the polyamide 6 oligomer in the polyamide 6 oligomer aqueous solution (B1) is not particularly limited, but it can be preferably exemplified that the content of a 2- to 12-mer linear polyamide oligomer is 90 mass% or more. It can be exemplified that the content of a linear 2- to 12-mer oligomer is more preferably 93 mass% or more, and particularly preferably 95 mass% or more. When the content of a 2- to 12-mer linear polyamide 6 oligomer contained in the polyamide 6 oligomer here is in such a preferable range, the solubility in water is increased, and further the concentration of the terminal carboxylic acid in the polyamide 6 oligomer is increased, so that the reaction of polyamide 6 with water is promoted, and the production efficiency of ε-caprolactam tends to increase. The amount of a linear 2- to 12-mer oligomer in the polyamide 6 oligomer here was quantitatively analyzed by high-performance liquid chromatography using a formic acid aqueous solution and a formic acid acetonitrile solution as eluents.

The method for preparing the polyamide 6 oligomer used in the polyamide 6 oligomer aqueous solution (B1) is not particularly limited, and for example, a polyamide 6 oligomer contained in an extract liquid when a polyamide 6 oligomer is extracted with hot water from a polyamide 6 resin at the time of production of a general fatty acid-based polyamide 6 resin, or a polyamide 6 oligomer prepared by the same method as a method for synthesizing a general fatty acid-based polyamide 6 resin may be used. In addition, a polyamide 6 oligomer obtained as a by-product when ε-caprolactam is produced by adding the resin composition (A) containing at least polyamide 6 and at least one of the water (B) heated to 290°C or higher and 350°C or lower and the polyamide 6 oligomer aqueous solution (B1) heated to 290°C or higher and 350°C or lower may be used. From the viewpoint of reducing an industrial waste in production of ε-caprolactam, it is preferable to use a polyamide 6 oligomer collected as a by-product when ε-caprolactam is produced by adding the resin composition (A) containing at least polyamide 6 and at least one of the water (B) heated to 290°C or higher and 350°C or lower and the polyamide 6 oligomer aqueous solution (B1) heated to 290°C or higher and 350°C or lower.

The polyamide 6 oligomer aqueous solution (B1) is prepared by heating and mixing the polyamide 6 oligomer and water. The water used here is not particularly limited, and tap water, ion exchanged water, distilled water, well water, or the like can be used, but ion exchanged water or distilled water is preferably used from the viewpoint of preventing side reactions due to the influence of coexisting salts. In addition, the concentration of the polyamide 6 oligomer in the polyamide 6 oligomer aqueous solution (B1) may be any concentration as long as the polyamide 6 oligomer is dissolved in water when heated to 290°C or higher and 350°C or lower, but can be exemplified by preferably 20 mass% or less, and is preferably 15 mass% or less, and can be more preferably exemplified by 10 mass% or less. When the concentration of the polyamide 6 oligomer in the polyamide 6 oligomer aqueous solution (B1) is in such a preferable range, solubility in water when the polyamide 6 oligomer aqueous solution (B1) is prepared is increased, and the polyamide 6 oligomer aqueous solution (B1) can be prepared at a lower temperature.

Further, the extract liquid containing a polyamide 6 oligomer obtained in the step of extracting a polyamide 6 oligomer with hot water from polyamide 6, which is a product at the time of production of polyamide 6, can also be used as the polyamide 6 oligomer aqueous solution (B1). Usually, a polyamide 6 resin obtained by polymerizing ε-caprolactam contains unreacted monomers generated in a polymerization equilibrium reaction and polyamide 6 oligomers as impurities. Therefore, in order to remove them, a pellet after polymerization is supplied to a hot water extraction tower, and unreacted monomers and polyamide 6 oligomers are extracted and removed by hot water extraction. Use of the extract liquid obtained in the step of extracting a polyamide 6 oligomer with hot water from polyamide 6 at the time of production of polyamide 6 as the polyamide 6 oligomer aqueous solution (B1) of the present invention can also be exemplified as a preferable form from the viewpoint of industrial waste reduction.

In the preparation of the mixture (C) of the present invention, various known reaction methods such as a batch method and a continuous method can be adopted. Examples of the batch method include an autoclave and a vertical/horizontal reactor, both having a stirrer and a heating function, and a vertical/horizontal reactor having a compression mechanism such as a cylinder in addition to a stirrer and a heating function. Examples of the continuous method include an extruder and a tubular reactor, both having a heating function, a tubular reactor having a mixing mechanism such as a baffle, a line mixer, a vertical/horizontal reactor, a vertical/horizontal reactor having a stirrer, and a tower. The atmosphere in the production is desirably a non-oxidizing atmosphere, preferably an inert atmosphere of nitrogen, helium, argon, or the like, and is preferably a nitrogen atmosphere from the viewpoint of economy and ease of handling.

### (4) Solid-liquid separation (I) step

The method for collecting a fibrous filling material (D) and ε-caprolactam of the present invention is characterized in that the mixture (C) containing at least the fibrous filling material (D), ε-caprolactam, the polyamide 6 oligomer, and water is subjected to solid-liquid separation (I) in a temperature range not higher than the boiling point of water at the operation pressure. By the solid-liquid separation (I) of the mixture (C) in a temperature range not higher than the boiling point of water at the operation pressure, ε-caprolactam and the polyamide 6 oligomer is separated into a liquid phase and the fibrous filling material (D) is separated into a solid phase. The solid-liquid separation (I) is not problematic at any temperature as long as it is in a temperature range not higher than the boiling point of water at the operation pressure, and the mixture (C) of the present invention is prepared at a temperature of 290°C or higher and 350°C or lower, and therefore, it is preferable to perform the solid-liquid separation (I) at a temperature not higher than the preparation temperature of the mixture (C). It can be exemplified that the solid-liquid separation (I) of the mixture (C) containing at least the fibrous filling material (D), ε-caprolactam, the polyamide 6 oligomer, and water is more preferably performed at 95°C or lower, and still more preferably performed at 90°C or lower.

Further, as a result of studying the dissolution behavior and precipitation behavior of the polyamide 6 oligomer in water in the present invention described in the above-mentioned item (2), the present inventors have found that the temperature at which the polyamide 6 oligomer is dissolved in water is 100°C or higher, whereas the temperature at which the polyamide 6 oligomer once dissolved in water is precipitated is lower than 100°C. The reason why the difference between the temperature at which the polyamide 6 oligomer is dissolved in water and the temperature at which the polyamide 6 oligomer is precipitated occurs in this manner that the polyamide 6 oligomer is in a supercooled state in a temperature range lower than 100°C and not lower than the temperature at which the polyamide 6 oligomer is precipitated. In general, in order to precipitate the dissolved polyamide 6 oligomer, the process goes through a stage in which first, a crystal nucleus of the polyamide 6 oligomer is formed, and the formed crystal nucleus grows to a sufficient size for precipitation to precipitate the polyamide 6 oligomer. Therefore, it is considered that, when the polyamide 6 oligomer aqueous solution dissolved in water is cooled, a crystal nucleus of the polyamide 6 oligomer is formed at a stage of cooling to lower than 100°C, which is a temperature at which the polyamide 6 oligomer is dissolved in water, and then the crystal nucleus formed in the cooling process grows to precipitate the polyamide 6 oligomer. From the above results, it can be preferably exemplified that the solid-liquid separation (I) of the mixture (C) containing at least the fibrous filling material, ε-caprolactam, the polyamide 6 oligomer, and water is preferably performed in a temperature range not higher than the boiling point at normal pressure, and it can be exemplified that the solid-liquid separation (I) is more preferably performed at 95°C or lower, and still more preferably performed at 90°C or lower. Further, in the solid-liquid separation (I), since the polyamide 6 oligomer is collected as a liquid phase component by solid-liquid separation, the lower limit temperature at which the solid-liquid separation (I) is performed may be a temperature at which the polyamide 6 oligomer is in a supercooled state, and can be specifically exemplified by preferably 50°C or higher, more preferably 60°C or higher, and still more preferably 70°C or higher. When the solid-liquid separation (I) is performed in such a preferable temperature range, at least 97% or more, preferably 98% or more, more preferably 99% or more of ε-caprolactam or the polyamide 6 oligomer is transformed into a collectable state as a liquid phase component, and there is a tendency that the collection loss of ε-caprolactam and the polyamide 6 oligomer by the solid-liquid separation (I) can be reduced.

Further, as the solid-liquid separation (I) of the mixture (C) containing at least the fibrous filling material (D), ε-caprolactam, the polyamide 6 oligomer, and water of the present invention, a method in which the mixture (C) separately prepared is reheated to a temperature at which the polyamide 6 oligomer is dissolved, cooled to the solid-liquid separation (I) temperature, and subjected to the solid-liquid separation (I), and a method in which the mixture (C) containing at least the fibrous filling material (D), ε-caprolactam, the polyamide 6 oligomer, and water is prepared by bringing the polyamide 6 resin composition (A) containing at least the fibrous filling material (D) into contact with the water (B) heated to 290°C or higher and 350°C or lower, and then subsequently cooled to the solid-liquid separation (I) temperature from the preparation temperature of the mixture (C) and subjected to the solid-liquid separation (I) can be mentioned. Preferably, a method in which the mixture (C) is prepared, and then subsequently cooled to a temperature at which the solid-liquid separation (I) is performed, and subjected to the solid-liquid separation (I) can be mentioned.

The method for performing the solid-liquid separation (I) is not particularly limited, and a known method can be adopted, and pressure filtration or vacuum filtration, which is filtration using a filter, centrifugation or precipitation separation, which is separation based on a difference in specific gravity between a solid component and a solution, a combination thereof, or the like can be adopted. A decanter separation method in which precipitation separation is performed before a filtration operation is also a preferable method. The filter used for the filtration operation may be any filter as long as it is stable under the conditions for performing the solid-liquid separation (I), and for example, a filter sieve or a sintered plate can be suitably used. In addition, the mesh diameter or pore diameter of the filter can be adjusted in a wide range depending on the viscosity, pressure, and temperature of the mixture (C) to be subjected to the filtration operation, the size of the fibrous filling material (D), the purity (solid component content) of the filtrate to be obtained, and the like. In particular, it is effective to select the mesh diameter or the pore diameter according to the size of the fibrous filling material (D) to be collected as a solid phase component by the solid-liquid separation (I) in the mixture (C) .

According to the solid-liquid separation (I) here, most of the fibrous filling material (D) in the mixture (C) can be separated as a filter residue, and preferably 95 mass% or more, more preferably 97 mass% or more, and still more preferably 99 mass% or more of the fibrous filling material (D) contained in the mixture (C) can be collected as a solid component. In addition, when the filter residue separated by the solid-liquid separation (I) contains water containing ε-caprolactam and the polyamide 6 oligomer, the amount of the ε-caprolactam and the polyamide 6 oligomer remaining in the filter residue can be reduced by washing the filter residue with fresh water (step (c)). The washing of the filter residue with fresh water here is performed in a temperature range not higher than the boiling point at normal pressure, and a temperature range of preferably 95°C or lower, more preferably 90°C or lower can be mentioned. The separation of the fibrous filling material (D) in the mixture (C) from ε-caprolactam and the polyamide 6 oligomer by the solid-liquid separation (I) and washing with water in this manner eliminates the need for a complicated process, and thus can be said to be a preferable method also from the viewpoint of process cost or environmental load.

### (5) Step of collecting fibrous filling material

In the present invention, the filter residue containing the fibrous filling material (D) obtained by the solid-liquid separation (I) is washed with water having a temperature not higher than the boiling point of water at normal pressure to collect the fibrous filling material (D) By washing the filter residue containing the fibrous filling material (D) with water having a temperature not higher than the boiling point of water at normal pressure, it is possible to collect the fibrous filling material (D) with high purity containing a small residual amount of organic substances. The temperature of water required for washing is not particularly limited, and may be not higher than the boiling point of water at normal pressure. Further, the collected fibrous filling material (D) is a fibrous filling material (D) with high purity containing a small residual amount of organic substances such as ε-caprolactam and a polyamide 6 oligomer. The mass loss when the collected fibrous filling material (D) is subjected to a heat treatment at 600°C for 3 hours in an air atmosphere can be preferably exemplified by 3.0 mass% or less, and more preferably 2.0 mass% or less. The fact that the mass loss of the collected fibrous filling material (D) during the heat treatment is in such a preferable range means that the amount of organic substances adhered to the fibrous filling material (D) is small, that is, it means that the collection loss of ε-caprolactam and the polyamide 6 oligomer in the solid-liquid separation (I) of the mixture (C) and the water washing step is small, and therefore, the mass loss in such a preferable range is preferable.

### (6) Method for collecting ε-caprolactam

The method for collecting ε-caprolactam from the filtrate obtained by the solid-liquid separation (I) of the present invention is not particularly limited, and any method can be adopted. For example, ε-caprolactam with high purity can be collected by performing a distillation operation of the ε-caprolactam aqueous solution obtained by the solid-liquid separation (I) to separate ε-caprolactam from water and the polyamide 6 oligomer. In addition, if a water-insoluble component is precipitated by cooling the ε-caprolactam aqueous solution collected by the solid-liquid separation (I), the water-insoluble component is separated in advance by a known method such as solid-liquid separation and can also be subjected to distillation separation.

Further, as a method for obtaining ε-caprolactam with high purity, it can be combined with a purification method such as a method for precisely distilling collected ε-caprolactam, a vacuum distillation method by adding a trace amount of sodium hydroxide, an activated carbon treatment method, an ion exchange treatment method, or a recrystallization method. By these methods, impurities that are difficult to separate by distillation separation can also be efficiently removed.

### (7) Polyamide 6 and molded product thereof

In the method for collecting ε-caprolactam described in the present invention, ε-caprolactam with high purity can be obtained, and therefore can be used as a polymerization raw material of polyamide 6. Polyamide 6 can be produced by a generally known method in which ε-caprolactam is subjected to hot melt polymerization in the presence of a small amount of water.

Further, the polyamide 6 thus obtained is melt-kneaded with the fibrous filling material (D) or various additives as necessary to produce a polyamide 6 resin composition, and various molded products such as a sheet and a film can be obtained by a generally known method such as injection molding or extrusion molding.

The polyamide 6 of the present invention and a molded product thereof can be used for various applications such as electrical/electronic parts, building members, various vessels, daily necessities, household goods, and sanitary goods by taking advantage of its excellent properties. In particular, it is particularly preferably used for aircraft parts and electrical/electronic parts which require toughness and stiffness. Specifically, aircraft-related parts such as a landing gear pod, a winglet, a spoiler, an edge, a ladder, an elevator, a fairing, and a rib, and as electrical/electronic parts, for example, electrical parts such as an electrical generator, an electric motor, a transformer, a current transformer, a voltage regulator, a rectifier, a resistor, an inverter, a relay, a power contact, an electrical switch, an interrupter, a switch, a knife switch, a multi-pole rod, a motor case, a TV housing, a laptop housing and internal parts, a CRT display housing and internal parts, a printer housing and internal parts, mobile terminal housings and internal parts such as a mobile phone, a mobile personal computer, and a handheld mobile terminal, IC- and LED-compatible housings, a capacitor plate, a fuse holder, various gears, various cases, and a cabinet, and electronic parts such as a connector, an SMT-compatible connector, a card connector, a jack, a coil, a coil bobbin, a sensor, an LED lamp, a socket, a resistor, a relay, a relay case, a reflector, a small switch, a power supply part, a coil bobbin, a capacitor, a variable capacitor case, an optical pickup chassis, an oscillator, various terminal boards, a transformer, a plug, a printed circuit board, a tuner, a speaker, a microphone, a headphone, a small motor, a magnetic head base, a power module, a Si power module and a SiC power module, a semiconductor, a liquid crystal, a FDD carriage, a FDD chassis, a motor brush holder, a transformer member, a parabolic antenna, and a computer-related part, and the like are mentioned.

### [Examples]

Hereinafter, the present invention will be described with reference to examples, but the present invention is not limited by these examples.

In each example, the following raw materials were used.

### [Polyamide 6 (PA6-A)]

Polyamide 6 resin ("Amilan" (registered trademark) CM1017 manufactured by Toray Industries, Inc.), ηr = 2.70, melting point: 225°C, amount of cyclic 2- to 4-mer oligomer: 0.2 mass%

Here, the solution viscosity ηr was measured at 25°C using a 0.01 g/mL solution of 98% concentrated sulfuric acid. Further, the melting point was defined as the temperature of an endothermic peak appearing when the polyamide was cooled from a molten state to 30°C at a cooling rate of 20°C/min and then heated to the melting point + 40°C at a heating rate of 20°C/min in a nitrogen gas atmosphere using a differential scanning calorimeter. However, when two or more endothermic peaks were detected, the temperature of the endothermic peak having a highest peak intensity was defined as the melting point.

Here, the amount of the cyclic 2- to 4-mer oligomer was determined by pulverizing polyamide 6, collecting polyamide 6 powder that passed through a JIS standard sieve of 24 mesh, but did not pass through a 124 mesh, subjecting 20 g of the polyamide 6 powder to extraction with 200 mL of methanol for 3 hours using a Soxhlet extractor, and quantitatively analyzing the cyclic oligomer contained in the extract liquid using high-performance liquid chromatography. The measurement conditions are as follows.

### <Measurement conditions>

High-performance liquid chromatograph: Waters' 600E
Column: GL Sciences' ODS-3
Detector: Waters' 484 Tunable Absorbance Detector
Detection wavelength: 254 nm
Solvent: methanol/water (the composition of methanol water is 20 : 80 → 80 : 20 gradient analysis)
Flow rate: 1 mL/min

[Fibrous filling material]

### Glass fibers (T-249 manufactured by Nippon Electric Glass Co., Ltd.)

### [Reference Example 1] Polyamide 6 including fibrous filling material (PA6-B)

Glass fiber-reinforced polyamide 6 (PA6-B) was prepared by blending the polyamide 6 (PA6-A) and glass fibers so that the mass ratio of the polyamide 6 to the glass fibers was 70/30, and supplying the polyamide 6 from a main feeder, supplying the glass fibers from a side feeder, and pelletizing the extruded strings using a twinscrew extruder (TEX30α manufactured by The Japan Steel Works, Ltd.) set at a cylinder set temperature of 250°C and a screw rotation speed of 150 rpm.

### [Reference Example 2] Waste of polyamide resin molded body (PA6-C)

A polyamide 6 resin containing 30 mass% of glass fibers ("Amilan" (registered trademark) CM1011G-30 manufactured by Toray Industries, Inc.) was molded into a dumbbell piece, and then crushed to obtain a polyamide 6 resin molded body waste in a pellet form.

### [Reference Example 3] Production of polyamide 6 oligomer

In an autoclave made of SUS316L equipped with a stirrer, 20.0 g of the polyamide 6 (PA6-A) and 60.0 g of deionized water were charged. The mass ratio (X : 1) of water to polyamide 6 is 3 : 1.

The reaction vessel was purged with nitrogen and sealed under a nitrogen pressure of 0.5 MPa, and then the reaction was carried out by holding the reaction vessel at 320°C for 15 minutes with stirring at 200 rpm. After completion of the reaction, the reaction mixture was cooled to room temperature and collected. Since the reaction temperature Y°C is 320°C, the product of X and Y is 960, and since the retention time at the reaction temperature of 320°C is 15 minutes, the product of X, Y, and Z is 14,400.

The yield of ε-caprolactam calculated by high-performance liquid chromatography measurement of the collected reaction mixture was 78%.

To the obtained reaction mixture, 10 times the amount (mass) of methanol was added, and the mixture was stirred to form a slurry, and then filtered through a glass filter (average pore diameter: 10 to 16 µm) to obtain a solid component. Further, an operation in which 5 times the amount of methanol is added to the obtained solid component, and the mixture was stirred to form a slurry, and then filtered through a glass filter was repeated 3 times, and then the filter residue was subjected to vacuum drying at 50°C for 12 hours to obtain a polyamide 6 oligomer.

The obtained polyamide 6 oligomer was subjected to high-performance liquid chromatography analysis and found that the amount of a linear 2- to 12-mer oligomer is 95.8 mass%. High performance liquid chromatography measurement conditions here are as follows.
Apparatus: LC-10Avp series manufactured by Shimadzu Corporation
Column: Mightysil RP-18GP 150-4.6
Detector: Photodiode array detector (UV = 205 nm)
Flow rate: 1 mL/min
Column temperature: 40°C
Mobile phase: 0.1% formic acid aqueous solution/0.1% formic acid acetonitrile solution
Polyamide 6 oligomer composition: the amount of a linear 2- to 12-mer oligomer in the polyamide 6 oligomer is calculated from the peak area ratio of each polyamide 6 oligomer

### [Reference Example 4] Hot water extraction of polyamide 6

In a 70-liter autoclave, 20 kg of ε-caprolactam, 4.32 g of benzoic acid, and 3.0 kg of ion exchanged water were charged, the inside of the polymerization can was sealed and sufficiently purged with nitrogen, and then the temperature was raised until the pressure inside the can of the polymerization reactor reached 0.98 MPa with stirring, and the temperature was continuously raised to 250°C while the pressure inside the can was maintained. After the temperature reached 250°C, the pressure was released to atmospheric pressure over 40 minutes. Thereafter, the mixture was held at 250°C for 180 minutes at atmospheric pressure, and then the polyamide 6 polymer was discharged and cooled/cut to form a pellet.

This pellet was subjected to extraction using 20 times the amount of hot water at 98°C to collect an extract liquid containing unreacted caprolactam and a polyamide 6 oligomer. The total amount of the unreacted caprolactam and the polyamide 6 oligomer in the extract liquid was 0.5 mass%, and the amount of the polyamide 6 oligomer was 0.1 mass%.

### <<Evaluation Method>>

### [Yield of ε-caprolactam (HPLC)]

The calculation of the yield of ε-caprolactam (HPLC) of the present invention was performed by high-performance liquid chromatography measurement. The measurement conditions are as follows.
Apparatus: LC-10Avp series manufactured by Shimadzu Corporation
Column: Mightysil RP-18GP 150-4.6
Detector: Photodiode array detector (UV = 205 nm)
Flow rate: 1 mL/min
Column temperature: 40°C
Mobile phase: 0.1% acetic acid aqueous solution/acetonitrile
Sample: A high-performance liquid chromatography analysis sample was prepared by taking about 0.1 g of the reaction mixture, diluting it with about 10 g of deionized water, and separating and removing components insoluble in deionized water by filtration.
Quantification of ε-caprolactam: The amount of ε-caprolactam with respect to polyamide 6 was quantified by an absolute calibration curve method.

### [Example 1]

In an autoclave made of SUS316L equipped with a stirrer, a bottom plug valve, and a glass filter (average opening: 10 µm) at the bottom, 28.6 g of PA6-B prepared in Reference Example 1 and 60.0 g of deionized water were charged. The mass ratio (X : 1) of water to polyamide 6 is 3 : 1.

The reaction vessel was purged with nitrogen and sealed under a nitrogen pressure of 0.5 MPa, and then the reaction was carried out by holding the reaction vessel at 320°C for 15 minutes with stirring at 200 rpm. The ultimate pressure during the reaction was 10.4 MPa. Since the reaction temperature Y°C is 320°C, the product of X and Y is 960, and since the retention time at the reaction temperature of 320°C is 15 minutes, the product of X, Y, and Z is 14,400.

After completion of the reaction, the internal temperature was cooled to 90°C, the bottom plug valve was opened while the temperature was maintained at 90°C to perform solid-liquid separation (I). The solid-liquid separation (I) was performed while nitrogen was introduced into the autoclave at 0.3 MPa at the stage when the filtration speed decreased. Further, about 3 times the amount (mass) of deionized water to the filter residue was introduced into the autoclave, and the filter residue was washed by rinsing at 90°C 3 times, and the filtrate and the wet filter residue were collected.

According to high-performance liquid chromatography measurement of the filtrate obtained by the solid-liquid separation (I), the amount of ε-caprolactam contained in the filtrate is 14.0 g, and the yield with respect to the polyamide 6 in PA6-B used as the raw material is 70.0%.

In addition, the obtained wet filter residue was subjected to vacuum drying at 50°C for 12 hours to collect 8.6 g of glass fibers. In a crucible, 1.0 g of the collected glass fibers were weighed and treated in an air atmosphere for 3 hours in an electric furnace heated to 600°C. The amount of organic substances adhered to the collected glass fibers was evaluated from the mass loss, and as a result, it was found that the mass loss was 1.4 mass%, and the collected glass fibers were glass fibers with high purity having a small amount of organic substances adhered thereto.

Further, the collected filtrate was heated to 55°C under a reduced pressure of 30 mmHg to separate water by distillation, thereby obtaining a concentrated ε-caprolactam aqueous solution, which was further distilled under a reduced pressure of 5 mmHg at a heating temperature of 150 to 170°C to collect distilled ε-caprolactam. The concentration and distillation yield was 95.8%. In addition, the HPLC impurity in the distilled ε-caprolactam was 0.48%, and the quality was such that it can be used also as a polymerization raw material of polyamide 6.

From the above, it can be seen that the present invention is a low environmental load process capable of collecting glass fibers and ε-caprolactam with high purity without using an organic solvent in a large amount.

### [Example 2]

In an autoclave made of SUS316L equipped with a stirrer, a bottom plug valve, and a glass filter (average opening: 10 µm) at the bottom, 28.6 g of the waste of a polyamide resin molded body PA6-C prepared in Reference Example 2 and 60.0 g of deionized water were charged. The mass ratio (X : 1) of water to polyamide 6 is 3 : 1.

The reaction vessel was purged with nitrogen and sealed under a nitrogen pressure of 0.5 MPa, and then the reaction was carried out by holding the reaction vessel at 340°C for 15 minutes with stirring at 200 rpm. Since the reaction temperature Y°C is 340°C, the product of X and Y is 1,020, and since the retention time at the reaction temperature of 340°C is 15 minutes, the product of X, Y, and Z is 15,300.

After completion of the reaction, the internal temperature was cooled to 80°C, the bottom plug valve was opened while the temperature was maintained at 80°C to perform solid-liquid separation (I). The solid-liquid separation (I) was performed while nitrogen was introduced into the autoclave at 0.3 MPa at the stage when the filtration speed decreased. Further, about 3 times the amount (mass) of deionized water to the filter residue was introduced into the autoclave, and the filter residue was washed by rinsing at 80°C 3 times, and the filtrate and the wet filter residue were collected.

According to high-performance liquid chromatography measurement of the filtrate obtained by the solid-liquid separation (I), the amount of ε-caprolactam contained in the filtrate is 14.2 g, and the yield with respect to the polyamide 6 in PA6-C used as the raw material is 70.9%.

In addition, the obtained wet filter residue was subjected to vacuum drying at 50°C for 12 hours to collect 8.6 g of glass fibers. In a crucible, 1.0 g of the collected glass fibers were weighed and treated in an air atmosphere for 3 hours in an electric furnace heated to 600°C. The amount of organic substances adhered to the collected glass fibers was evaluated from the mass loss, and as a result, it was found that the mass loss was 1.5 mass%, and the collected glass fibers were glass fibers with high purity having a small amount of organic substances adhered thereto.

### [Example 3]

In an autoclave made of SUS316L equipped with a stirrer, a bottom plug valve, and a glass filter (average opening: 10 µm) at the bottom, 25.1 g of PA6-B prepared in Reference Example 1 and 25.5 g of deionized water were weighed, and 36.9 g of a polyamide 6 oligomer aqueous solution having a concentration of 6.5 mass% was further added thereto. The mass ratio of water to the sum of polyamide 6 and the polyamide 6 oligomer is as follows: X : 1 = 3 : 1. The polyamide 6 oligomer here is a polyamide 6 oligomer produced by the method described in Reference Example 3.

The reaction vessel was purged with nitrogen and sealed under a nitrogen pressure of 0.5 MPa, and then the reaction was carried out by holding the reaction vessel at 320°C for 15 minutes with stirring at 200 rpm. Since the reaction temperature Y°C is 320°C, the product of X and Y is 960, and since the retention time at the reaction temperature of 320°C is 15 minutes, the product of X, Y, and Z is 14,400.

After completion of the reaction, the internal temperature was cooled to 90°C, the bottom plug valve was opened while the temperature was maintained at 90°C to perform solid-liquid separation (I). The solid-liquid separation (I) was performed while nitrogen was introduced into the autoclave at 0.3 MPa at the stage when the filtration speed decreased. Further, about 3 times the mass of deionized water to the filter residue was introduced into the autoclave, and the filter residue was washed by rinsing at 90°C 3 times, and the filtrate and the wet filter residue were collected.

According to high-performance liquid chromatography measurement of the obtained filtrate, the amount of ε-caprolactam contained in the filtrate was 14.5 g, and the yield with respect to the polyamide 6 in PA6-B used as the raw material was 82.5%.

In addition, the obtained wet filter residue was subjected to vacuum drying at 50°C for 12 hours to collect 7.5 g of glass fibers. In a crucible, 1.0 g of the collected glass fibers were weighed and treated in an air atmosphere for 3 hours in an electric furnace heated to 600°C, and the amount of organic substances adhered to the collected glass fibers was evaluated from the mass loss, and as a result, it was found that the mass loss was 1.2 mass%, and the collected glass fibers were glass fibers with high purity having a small amount of organic substances adhered thereto.

### [Comparative Example 1]

In an autoclave made of SUS316L equipped with a stirrer, 28.6 g of PA6-B prepared in Reference Example 1 and 60.0 g of deionized water were charged. The mass ratio (X : 1) of water to polyamide 6 is 3 : 1.

The reaction vessel was purged with nitrogen and sealed under a nitrogen pressure of 0.5 MPa, and then the reaction was carried out by holding the reaction vessel at 320°C for 15 minutes with stirring at 200 rpm, followed by cooling to prepare the reaction mixture. Since the reaction temperature Y°C is 320°C, the product of X and Y is 960, and since the retention time at the reaction temperature of 320°C is 15 minutes, the product of X, Y, and Z is 14,400.

According to high-performance liquid chromatography measurement of the obtained reaction mixture, the amount of ε-caprolactam contained in the reaction mixture was 14.0 g, and the yield with respect to the polyamide 6 in PA6-B used as the raw material was 70.0%.

Further, the reaction mixture was heated to 55°C under a reduced pressure of 30 mmHg to separate water by distillation, thereby obtaining a concentrated ε-caprolactam aqueous solution, which was further distilled under a reduced pressure of 5 mmHg at a heating temperature of 150 to 170°C to collect distilled ε-caprolactam. Meanwhile, an attempt was made to collect glass fibers from the distillation residue, but since a solvent-insoluble component was generated by long-time high-temperature heating during distillation, organic residues could not be separated by solid-liquid separation, and it was difficult to collect glass fibers with high purity.

Comparison between Example 1 and Comparative Example 1 shows that performing the solid-liquid separation (I) subsequently to the reaction between the polyamide 6 resin composition containing a fibrous filling material and water is a low environmental load process in which ε-caprolactam with high purity and glass fibers with high purity having a small amount of organic substances adhered thereto can be collected, and an industrial waste can be reduced.

### [Example 4] Polymerization of polyamide 6

In a test tube, 10 g of ε-caprolactam collected by the method described in Example 1, 2.2 mg of benzoic acid, and 10.0 g of ion exchanged water were weighed. The test tube was placed in an autoclave, the inside of the autoclave was purged with nitrogen, and then the jacket temperature was set to 250°C, and heating was started. After the internal pressure reached 1.0 MPa, the internal pressure was maintained at 1.0 MPa for 3 hours. Thereafter, the internal pressure was released to normal pressure over 1.5 hours, and heating was stopped when the internal temperature reached 228°C. After completion of the polymerization, the polymer was collected from the test tube and subjected to a crushing treatment. The crushed polymer was treated in hot water at 95°C for 15 hours to extract and remove unreacted monomers and low polymers. The polymer after extraction was subjected to vacuum drying at 80°C for 24 hours to obtain a polyamide 6 resin having a melting point of 226°C and ηr = 2.73. The solution viscosity ηr here was measured at 25°C using a 0.01 g/mL solution of 98% concentrated sulfuric acid.

### [Example 5]

In an autoclave made of SUS316L equipped with a stirrer, a bottom plug valve, and a glass filter (average opening: 10 µm) at the bottom, 58.0 g of PA6-B prepared in Reference Example 1 and 122.0 g of deionized water were weighed. The mass ratio (X : 1) of water to polyamide 6 is 3 : 1.

The reaction vessel was purged with nitrogen and sealed under a nitrogen pressure of 5.0 MPa, and then the reaction was carried out by holding the reaction vessel at 320°C for 15 minutes with stirring at 200 rpm. The ultimate pressure during the reaction was 19.7 MPa. Since the reaction temperature Y°C is 320°C, the product of X and Y is 960, and since the retention time at the reaction temperature of 320°C is 15 minutes, the product of X, Y, and Z is 14,400.

After completion of the reaction, the internal temperature was cooled to 90°C, the bottom plug valve was opened while the temperature was maintained at 90°C to perform solid-liquid separation (I). The solid-liquid separation (I) was performed while nitrogen was introduced into the autoclave at 0.3 MPa at the stage when the filtration speed decreased. Further, about 3 times the amount (mass) of deionized water to the filter residue was introduced into the autoclave, and the filter residue was washed by rinsing at 90°C 3 times, and the filtrate and the wet filter residue were collected.

According to high-performance liquid chromatography measurement of the filtrate obtained by the solid-liquid separation (I), the amount of ε-caprolactam contained in the filtrate was 32.9 g, and the yield with respect to the polyamide 6 in PA6-B used as the raw material was 81.0%.

In addition, the obtained wet filter residue was subjected to vacuum drying at 50°C for 12 hours to collect 17.4 g of glass fibers. In a crucible, 1.0 g of the collected glass fibers were weighed and treated in an air atmosphere for 3 hours in an electric furnace heated to 600°C. The amount of organic substances adhered to the collected glass fibers was evaluated from the mass loss, and as a result, it was found that the mass loss was 1.3 mass%, and the collected glass fibers were glass fibers with high purity having a small amount of organic substances adhered thereto.

Comparison with Example 1 shows that there is a tendency that ε-caprolactam is obtained in high yield by setting the pressure during the reaction to a high pressure equal to or higher than the saturated vapor pressure.

### [Example 6]

In an autoclave made of SUS316L equipped with a stirrer, a bottom plug valve, and a glass filter (average opening: 10 µm) at the bottom, 43.0 g of PA6-B prepared in Reference Example 1 and 60.0 g of deionized water were weighed. The mass ratio (X : 1) of water to polyamide 6 is 2 : 1.

The reaction vessel was purged with nitrogen and sealed under a nitrogen pressure of 0.3 MPa, and then the reaction was carried out by holding the reaction vessel at 320°C for 15 minutes with stirring at 200 rpm. Since the reaction temperature Y°C is 320°C, the product of X and Y is 640, and since the retention time at the reaction temperature of 320°C is 15 minutes, the product of X, Y, and Z is 9,600.

After completion of the reaction, the internal temperature was cooled to 90°C, the bottom plug valve was opened while the temperature was maintained at 90°C to perform solid-liquid separation (I). The solid-liquid separation (I) was performed while nitrogen was introduced into the autoclave at 0.3 MPa at the stage when the filtration speed decreased. Further, about 3 times the amount (mass) of deionized water to the filter residue was introduced into the autoclave, and the filter residue was washed by rinsing at 90°C 3 times, and the filtrate and the wet filter residue were collected.

According to high-performance liquid chromatography measurement of the filtrate obtained by the solid-liquid separation (I), the amount of ε-caprolactam contained in the filtrate was 19.2 g, and the yield with respect to the polyamide 6 in PA6-B used as the raw material was 63.9%.

In addition, the obtained wet filter residue was subjected to vacuum drying at 50°C for 12 hours to collect 12.9 g of glass fibers. In a crucible, 1.0 g of the collected glass fibers were weighed and treated in an air atmosphere for 3 hours in an electric furnace heated to 600°C. The amount of organic substances adhered to the collected glass fibers was evaluated from the mass loss, and as a result, it was found that the mass loss was 1.4 mass%, and the collected glass fibers were glass fibers with high purity having a small amount of organic substances adhered thereto.

### [Example 7]

In an autoclave made of SUS316L equipped with a stirrer, a bottom plug valve, and a glass filter (average opening: 10 µm) at the bottom, 75.0 g of PA6-B prepared in Reference Example 1 and 150.0 g of deionized water were weighed. The mass ratio (X : 1) of water to polyamide 6 is 2 : 1.

The reaction vessel was purged with nitrogen and sealed under a nitrogen pressure of 5.0 MPa, and then the reaction was carried out by holding the reaction vessel at 320°C for 15 minutes with stirring at 200 rpm. The ultimate pressure during the reaction was 19.8 MPa. Since the reaction temperature Y°C is 320°C, the product of X and Y is 640, and since the retention time at the reaction temperature of 320°C is 15 minutes, the product of X, Y, and Z is 9,600.

After completion of the reaction, the internal temperature was cooled to 90°C, the bottom plug valve was opened while the temperature was maintained at 90°C to perform solid-liquid separation (I). The solid-liquid separation (I) was performed while nitrogen was introduced into the autoclave at 0.3 MPa at the stage when the filtration speed decreased. Further, about 3 times the amount (mass) of deionized water to the filter residue was introduced into the autoclave, and the filter residue was washed by rinsing at 90°C 3 times, and the filtrate and the wet filter residue were collected.

According to high-performance liquid chromatography measurement of the filtrate obtained by the solid-liquid separation (I), the amount of ε-caprolactam contained in the filtrate was 39.3 g, and the yield with respect to the polyamide 6 in PA6-B used as the raw material was 74.9%.

Comparison with Example 6 shows that there is a tendency that ε-caprolactam is obtained in high yield by setting the pressure during the reaction to a high pressure equal to or higher than the saturated vapor pressure.

In addition, the obtained wet filter residue was subjected to vacuum drying at 50°C for 12 hours to collect 22.5 g of glass fibers. In a crucible, 1.0 g of the collected glass fibers were weighed and treated in an air atmosphere for 3 hours in an electric furnace heated to 600°C. The amount of organic substances adhered to the collected glass fibers was evaluated from the mass loss, and as a result, it was found that the mass loss was 1.4 mass%, and the collected glass fibers were glass fibers with high purity having a small amount of organic substances adhered thereto.

### [Example 8]

Here, an example, in which the hot water extract liquid in the PA6 production step obtained by the method described in Reference Example 4 was concentrated until the concentration of unreacted caprolactam and the polyamide 6 oligomer reached 6.5 mass%, and used as a depolymerization raw material, is described.

In an autoclave made of SUS316L equipped with a stirrer, a bottom plug valve, and a glass filter (average opening: 10 µm) at the bottom, 25.1 g of PA6-B prepared in Reference Example 1 and 25.5 g of deionized water were weighed, and 36.9 g of the polyamide 6 oligomer aqueous solution having a concentration of 6.5 mass% prepared above was further added thereto. The mass ratio of water to the sum of polyamide 6 and the polyamide 6 oligomer is as follows: X : 1 = 3.3 : 1.

The reaction vessel was purged with nitrogen and sealed under a nitrogen pressure of 0.5 MPa, and then the reaction was carried out by holding the reaction vessel at 320°C for 15 minutes with stirring at 200 rpm. Since the reaction temperature Y°C is 320°C, the product of X and Y is 1,056, and since the retention time at the reaction temperature of 320°C is 15 minutes, the product of X, Y, and Z is 15,840.

After completion of the reaction, the internal temperature was cooled to 90°C, the bottom plug valve was opened while the temperature was maintained at 90°C to perform solid-liquid separation (I). The solid-liquid separation (I) was performed while nitrogen was introduced into the autoclave at 0.3 MPa at the stage when the filtration speed decreased. Further, about 3 times the mass of deionized water to the filter residue was introduced into the autoclave, and the filter residue was washed by rinsing at 90°C 3 times, and the filtrate and the wet filter residue were collected.

According to high-performance liquid chromatography measurement of the obtained filtrate, the amount of ε-caprolactam contained in the filtrate was 14.0 g, and the yield with respect to the polyamide 6 in PA6-B used as the raw material was 79.7%.

In addition, the obtained wet filter residue was subjected to vacuum drying at 50°C for 12 hours to collect 7.5 g of glass fibers. In a crucible, 1.0 g of the collected glass fibers were weighed and treated in an air atmosphere for 3 hours in an electric furnace heated to 600°C, and the amount of organic substances adhered to the collected glass fibers was evaluated from the mass loss, and as a result, it was found that the mass loss was 1.2 mass%, and the collected glass fibers were glass fibers with high purity having a small amount of organic substances adhered thereto.

### [Example 9]

Here, an example, in which the hot water extract liquid in the PA6 production step obtained by the method described in Reference Example 4 was concentrated until the total concentration of unreacted caprolactam and the polyamide 6 oligomer reached 6.5 mass%, and used as a depolymerization raw material, is described.

In an autoclave made of SUS316L equipped with a stirrer, a bottom plug valve, and a glass filter (average opening: 10 µm) at the bottom, 25.5 g of PA6-B prepared in Reference Example 1 and 60.0 g of the polyamide 6 oligomer aqueous solution having a concentration of 6.5 mass% prepared above were added. The mass ratio of water to the sum of polyamide 6 and the polyamide 6 oligomer is as follows: X : 1 = 3.0 : 1.

The reaction vessel was purged with nitrogen and sealed under a nitrogen pressure of 0.5 MPa, and then the reaction was carried out by holding the reaction vessel at 320°C for 15 minutes with stirring at 200 rpm. Since the reaction temperature Y°C is 320°C, the product of X and Y is 963, and since the retention time at the reaction temperature of 320°C is 15 minutes, the product of X, Y, and Z is 14,454.

After completion of the reaction, the internal temperature was cooled to 90°C, the bottom plug valve was opened while the temperature was maintained at 90°C to perform solid-liquid separation (I). The solid-liquid separation (I) was performed while nitrogen was introduced into the autoclave at 0.3 MPa at the stage when the filtration speed decreased. Further, about 3 times the mass of deionized water to the filter residue was introduced into the autoclave, and the filter residue was washed by rinsing at 90°C 3 times, and the filtrate and the wet filter residue were collected.

According to high-performance liquid chromatography measurement of the obtained filtrate, the amount of ε-caprolactam contained in the filtrate was 14.0 g, and the yield with respect to the polyamide 6 in PA6-B used as the raw material was 78.4%.

In addition, the obtained wet filter residue was subjected to vacuum drying at 50°C for 12 hours to collect 7.6 g of glass fibers. In a crucible, 1.0 g of the collected glass fibers were weighed and treated in an air atmosphere for 3 hours in an electric furnace heated to 600°C, and the amount of organic substances adhered to the collected glass fibers was evaluated from the mass loss, and as a result, it was found that the mass loss was 1.3 mass%, and the collected glass fibers were glass fibers with high purity having a small amount of organic substances adhered thereto.

## Claims

1. A method for collecting a fibrous filling material and ε-caprolactam, comprising
adding a polyamide 6 resin composition (A) containing at least a fibrous filling material (D) and at least one of water (B) heated to 290°C or higher and 350°C or lower and a polyamide 6 oligomer aqueous solution (B1) heated to 290°C or higher and 350°C or lower to make contact with each other to obtain a mixture (C) containing at least the fibrous filling material (D), ε-caprolactam, a polyamide 6 oligomer, and the water (B), and
collecting the fibrous filling material (D) and the ε-caprolactam from the mixture (C), wherein steps (a) to (c) below are performed in this order:
(a) a step of preparing the mixture (C) by adding the polyamide 6 resin composition (A) containing the fibrous filling material (D) and at least one of the water (B) and the polyamide 6 oligomer aqueous solution (B1) to make contact with each other under a condition that a product of X and Y is 2,000 or less when a mass ratio of water to polyamide 6 or a mass ratio of water to a sum of polyamide 6 and the polyamide 6 oligomer is represented by X : 1 and a reaction temperature is represented by Y°C;
(b) a step of subjecting the mixture (C) to a solid-liquid separation (I) in a temperature range not higher than a boiling point of water at an operation pressure; and
(c) a step of washing a filter residue containing the fibrous filling material (D) obtained by the solid-liquid separation (I) with water having a temperature not higher than the boiling point of water at normal pressure to collect the fibrous filling material (D).

2. The method for collecting a fibrous filling material and ε-caprolactam according to claim 1, in which the polyamide 6 resin composition (A) containing at least the fibrous filling material (D) and the water (B) heated to 290°C or higher and 350°C or lower, or, the water (B) heated to 290°C or higher and 350°C or lower and the polyamide 6 oligomer aqueous solution (B1) heated to 290°C or higher and 350°C or lower are added to make contact with each other to obtain the mixture (C) containing at least the fibrous filling material (D), the ε-caprolactam, the polyamide 6 oligomer, and the water (B), and then the fibrous filling material (D) and the ε-caprolactam are collected from the mixture (C), wherein steps (a) to (c) below are performed in this order:
(a) a step of preparing the mixture (C) by adding the polyamide 6 resin composition (A) containing the fibrous filling material (D) and the water (B), or by adding the polyamide 6 resin composition (A) containing the fibrous filling material (D), the water (B), and the polyamide 6 oligomer aqueous solution (B1), to make contact with each other under a condition that a product of X and Y is 2,000 or less when a mass ratio of water to polyamide 6 or a mass ratio of water to a sum of polyamide 6 and the polyamide 6 oligomer is represented by X : 1 and a reaction temperature is represented by Y°C;
(b) a step of subjecting the mixture (C) to solid-liquid separation (I) in a temperature range not higher than a boiling point of water at an operation pressure; and
(c) a step of washing a filter residue containing the fibrous filling material (D) obtained by the solid-liquid separation (I) with water having a temperature not higher than the boiling point of water at normal pressure to collect the fibrous filling material (D).

3. The method for collecting a fibrous filling material and ε-caprolactam according to claim 1 or 2, wherein a mass loss of the fibrous filling material (D) when the fibrous filling material (D) collected is subjected to a heating treatment in an air atmosphere at 600°C for 3 hours is 3.0 mass% or less.

4. The method for collecting a fibrous filling material and ε-caprolactam according to any one of claims 1 to 3, wherein the solid-liquid separation (I) is performed in a temperature range not higher than the boiling point of water at normal pressure.

5. The method for collecting a fibrous filling material and ε-caprolactam according to any one of claims 1 to 4, wherein the mixture (C) containing at least the fibrous filling material (D), the ε-caprolactam, the polyamide 6 oligomer, and water is prepared, and then subsequently subjected to the solid-liquid separation (I).

6. The method for collecting a fibrous filling material and ε-caprolactam according to any one of claims 1 to 5, wherein the polyamide 6 oligomer aqueous solution (B1) is an extract liquid obtained in a step of extracting a polyamide 6 oligomer with hot water from polyamide 6 which is a product at a time of production of polyamide 6.

7. The method for collecting a fibrous filling material and ε-caprolactam according to any one of claims 1 to 6, wherein the polyamide 6 resin composition (A) containing at least the fibrous filling material (D) is a waste of a resin molded body containing polyamide 6 including at least the fibrous filling material (D).

8. A method for producing polyamide 6, comprising obtaining ε-caprolactam by the method according to any one of claims 1 to 7, and polymerizing polyamide 6.
